# EUROPÄISCHE PATENTSCHRIFT

(11) **EP 3 320 320 B1**
(45) Veröffentlichungstag und Bekanntmachung des Hinweises auf die Patenterteilung: **13.04.2022**
(21) Anmeldenummer: 16734257.5
(22) Anmeldetag: 27.06.2016
(51) Int. Cl.: G01N 1/22

(54) **FLÜSSIGKEITSGEKÜHLTE GASENTNAHMESONDE**
LIQUID-COOLED GAS SAMPLING PROBE
SONDE DE PRÉLÈVEMENT DE GAZ À REFROIDISSEMENT LIQUIDE

(30) Priorität: 06.07.2015 DE 102015110873; 18.12.2015 DE 102015122251
(43) Veröffentlichungstag der Anmeldung: 16.05.2018
(73) Patentinhaber: Enotec GmbH Prozess und Umweltmesstechnik, 51709 Marienheide (DE)
(72) Erfinder: GUMPRECHT, Fred, 51709 Marienheide (DE)
(74) Vertreter: Freischem & Partner Patentanwälte mbB
(86) Internationale Anmeldenummer: PCT/EP2016/064893
(87) Internationale Veröffentlichungsnummer: WO 2017/005521

(56) Entgegenhaltungen:
- EP-A1- 1 371 976
- WO-A1-95/28628
- GB-A- 1 340 918
- US-A- 4 161 883
- US-A- 4 772 454
- US-A1- 2005 084 976
- US-A1- 2005 241 416

## Beschreibung

Die Erfindung betrifft eine flüssigkeitsgekühlte Gasentnahmesonde mit:
- einem von Kühlflüssigkeit durchströmten Sondenrohr mit einem Außenmantel und einem Innenmantel, die dicht miteinander verbunden sind und die Begrenzung eines Strömungsraums bilden,
- mindestens einem rohrförmigen Filter aus Sinterwerkstoff, der innerhalb des Innenmantels des Sondenrohrs angeordnet ist, wobei durch das freie Ende des Sondenrohrs in den Innenmantel des Sondenrohrs eintretendes Messgas den rohrförmigen Filter von außen nach innen durchströmt,
- mindestens einer Messkammer mit mindestens einem Messsensor, in die das Messgas nach dem Durchströmen des rohrförmigen Filters eintritt,
- einer Ausstoßplatte, welche im Bereich des freien Endes des Sondenrohrs angeordnet und innerhalb des Innenmantels axial verschiebbar ist, wobei im Messbetrieb ein schmaler Ringspalt zwischen der Außenseite der Ausstoßplatte und dem Innenmantel des Sondenrohrs das Einströmen von Messgas in das Sondenrohr ermöglicht.

Eine derartige Gasentnahmesonde ist aus der Druckschrift WO 95/28628 A1 oder der Druckschrift EP 1 371 976 B1 bekannt. Die Gasentnahmesonde aus der EP 1 371 976 B1 ist dazu bestimmt, den Betreibern der Feuerungsanlagen von Kraftwerken, Müllverbrennungsanlagen, Glasschmelzen, Zementdrehöfen und ähnlichen Anlagen eine einwandfreie Verbrennung durch Regelung der Feuerung zu gewährleisten. Um die vorgeschriebenen Grenzwerte für die Abgaszusammensetzung einhalten zu können, ist ständig die Zusammensetzung und insbesondere die Sauerstoffkonzentration im Rauchgas zu überwachen. Als Kühlflüssigkeit wird in aller Regel Wasser, ggf. mit einem Frostschutzmittel oder anderen Additiven verwendet. Aus diesem Grund wird in der Folge auch von Wasserkühlung gesprochen. Es ist aber auch möglich, eine andere Kühlflüssigkeit, z.B. Öl, zu verwenden, die einen anderen Siedepunkt aufweist, als Wasser. Ein Zwischenmantel zwischen Außenmantel und Innenmantel teilt den Strömungsraum für die Kühlflüssigkeit in einen äußeren Zuströmraum mit ringförmigem Querschnitt, in dem Kühlflüssigkeit von einem kalten Ende des Sondenrohrs zu dessen freien Ende innerhalb des Rauchgases leitet, und in einen inneren Abströmraum mit ringförmigem Querschnitt, in dem die Kühlflüssigkeit von dem freien Ende zum kalten Ende des Sondenrohrs zurückströmt. Es sind aber auch andere Strukturen zum Leiten der Strömung der Kühlflüssigkeit zwischen Außenmantel und Innenmantel möglich, zum Beispiel Rohre mit kleinem Durchmesser.

Frühere Messverfahren und Messanordnungen zur Bestimmung der gasförmigen Rauchgasbestandteile schlugen vor, mittels wassergekühlter Gasentnahmesonden das Messgas aus dem Feuerraum anzusaugen und über temperierte Schläuche und weite Wege einem Gas-Analysengerät zuzuführen. Das Rauchgas brauchte mehrere Minuten, um von dem Feuerraum zum Analysegerät zu gelangen. Die geänderte Gaszusammensetzung konnte also nur mit einer großen zeitlichen Verzögerung gemessen werden. In stark staubbeladenen Feuerungen von z. B. Zementdrehöfen bestand bei den zuvor bekannten Sonden die Gefahr des Anbackens von Staub im Innern des Messraumes (Rohmehlansatz).

Diese Nachteile wurden durch die Gasentnahmesonde gemäß der Druckschrift EP 1 371 976 B1 verringert oder beseitigt. Der Messsensor befindet sich innerhalb eines Innenmantels des wassergekühlten Sondenrohrs nahe der Entnahmestelle. Das entnommene Gas durchströmt einen aus einem Sinterwerkstoff gefertigten, rohrförmigen Filter, der eine Messkammer umschließt, so dass Staubpartikel vor Erreichen des Messsensors aus dem Rauchgas herausgefiltert werden. Mit der Messkammer ist eine Ausstoßplatte verbunden, die sich innerhalb des Innenmantels nahe dem freien Ende des Sondenrohrs befindet. Die Ausstoßplatte ist innerhalb des Innenmantels axial beweglich. Zwischen der Ausstoßplatte und dem Innenmantel ist ein Ringspalt vorgesehen, durch den das Messgas in das Sondenrohr einströmt und zum Filter hin strömt. Die Messkammer mit dem Filter und der Ausstoßplatte ist axial verschiebbar innerhalb des Innenmantels angeordnet. Sie wird in regelmäßigen Abständen axial nach vorne geschoben, damit die Ausstoßplatte eine Ansammlung von Partikeln am freien Ende des Sondenrohrs innerhalb des Feuerraums beseitigt. Das Sondenrohr selbst wird in regelmäßigen Zeitabständen um einen Winkel von 90° gedreht. Auf diese Weise soll erreicht werden, dass Ablagerungen von Partikeln an dem Sondenrohr herunterfallen und das Sondenrohr durch derartige Ablagerungen nicht fest mit der Eintrittsöffnung des Feuerraums verbunden wird, durch welche das Sondenrohr ragt.

Die Druckschrift GB 1 340 918 A offenbart eine Gasanalysevorrichtung, bei der ein Filter in einem Kühlrohr angeordnet ist. An der Spitze einer Entstaubungsstange 3 sind mehrere schalenförmige Metallteile oder Bürsten zum Abkratzen von auf dem Filter abgelagertem Staub angeordnet. Die Druckschrift US 4 772 454 A beschreibt eine Sonde zur Entnahme staubigen Gases mit einem Kühlmantel und einem Kanal, der zu einer Kammer mit einem Filter führt. Der Kanaleinlass ist konvergent, um die Gasgeschwindigkeit an der Einlassöffnung und damit die Staubbelastung zu reduzieren. Beim Einströmen in den Kanaleinlass wird das Gas auf eine höhere Geschwindigkeit beschleunigt. Die Druckschrift US 4 161 883 A offenbart dagegen eine Gasentnahmesonde mit einem Sammelrohr, das Probengas aus einem haubenförmigen Filter innerhalb eines zylindrischen Schutzrohres ansaugt und zu einem inneren Filter transportiert.

Die Gasentnahmesonde der EP 1 371 976 B1 hat sich in der Praxis bewährt. Allerdings wurden bei Ihrem Betrieb Probleme aufgrund von Partikelablagerungen beobachtet, welche durch die vorliegende Erfindung beseitigt werden sollen. Insbesondere konnte nach einer gewissen Betriebszeit der rohrförmige Filter verstopfen. Auch konnte durch das Drehen des Sondenrohrs das Anbacken von Partikeln nicht immer zuverlässig vermieden werden.

Die vorliegende Erfindung soll diese Probleme beseitigen.

Zu diesem Zweck wird vorgeschlagen, zwischen dem Filter und der Ausstoßplatte ein Verbindungselement anzuordnen, dessen Durchmesser mehrere Millimeter kleiner ist als der Innendurchmesser des Innenmantels, so dass zwischen dem Verbindungselement und dem Innenmantel ein Ringraum entsteht, dessen radiale Erstreckung einem Mehrfachen der radialen Erstreckung des Ringspalts zwischen Ausstoßplatte und Innenmantel entspricht.

Wie bereits bei der bekannten Messsonde der EP 1 371 976 B1 wird durch die ringförmige Einströmöffnung für das Messgas im Bereich des freien Endes des Sondenrohrs ein großer Teil der Partikel außerhalb des Sondenrohrs gehalten. Bei der vorliegenden Entwicklung ist aber ein Verbindungselement zwischen der Ausstoßplatte und dem Filter, welches einen bedeutend geringeren Durchmesser als der Innenmantel des Sondenrohrs aufweist. Hierdurch wird ein großvolumiger Ringraum im Anschluss an den Ringspalt geschaffen, in dem die Strömungsgeschwindigkeit des durch den Ringspalt eintretenden Messgases erheblich verlangsamt wird. Während des Durchströmens des Ringraums lagern sich in dem Messgas vorhandene Partikel aufgrund der Schwerkraft in den Ringraum ab, so dass sie den Filter nicht erreichen. Wenn durch die Ausgestaltung des Ringspaltes dem Messgas eine Geschwindigkeitskomponente in Umfangsrichtung des Sondenrohrs aufgeprägt wird, findet zusätzlich zu der Ablagerung durch Schwerkraft noch eine Ablagerung aufgrund eines Zykloneffekts in dem Ringraum statt, das heißt, die Partikel werden durch Zentrifugalkraft der sich um die Achse des Sondenrohrs drehenden Gasströmung zur Wand des Ringraums bewegt. An den Filter gelangt nur noch ein ganz geringer Anteil der Partikel. Folglich ist die Gefahr des Verstopfens der Filteroberfläche durch Partikel erheblich reduziert.

Wie bei der bekannten Messsonde wird der Strömungsweg des Messgases regelmäßig gespült. Zu diesem Zweck wird ein Spülgas durch den Innenmantel geleitet, welches die Filteroberfläche mit hoher Strömungsgeschwindigkeit überströmt und durch den Ringraum und den Ringspalt aus dem freien Ende des Sondenrohrs herausströmt. Beim Überströmen der Filteroberfläche und beim Durchströmen des Ringraums und des Ringspalts reißt das Spülgas abgelagerte Partikel mit. Während des Spülens wird die Ausstoßplatte aus dem freien Ende des Sondenrohrs herausbewegt, damit Partikel, die sich auf der Stirnseite des Sondenrohrs im Bereich des Ringspalts abgelagert haben, losgelöst und abgestoßen werden. Die Austrittsöffnung für das Spülgas ist bei vollständig herausbewegter Ausstoßplatte sehr viel größer als der Ringspalt. Durch den Ringraum mit großem Querschnitt ist der Volumenstrom an Spülgas während des Rückspülens viel größer als bei der bekannten Sonde mit einem kleinen Ringspalt. Aufgrund des großen Volumenstroms an Spülgas werden sämtliche auf der Filteroberfläche und im Ringraum befindlichen Partikel ausgeblasen. Zudem bildet sich zu Beginn des Spülvorgangs ein Druckstoß aus, der zusätzlich das Loslösen von Ablagerungen begünstigt. Durch den vergrößerten Volumenstrom an Spülgas, der über die Filteroberfläche nach außen strömt sowie durch die verringerte Ablagerung von Partikeln auf dem Filter selbst wird vermieden, dass dauerhafte Ablagerungen auf der Filteroberfläche entstehen und dessen Durchlässigkeit verringern.

Die erfindungsgemäße Messsonde kann ohne Störungen der Messgasentnahme und ohne Notwendigkeit manueller Reinigungsarbeiten über einen jahrelangen Zeitraum betrieben werden. Sie liefert zuverlässig die erforderlichen Insitu-Messergebnisse der Rauchgaszusammensetzung, ohne dass für Wartungsarbeiten oder Reinigungsarbeiten an der Gasentnahmesonde der Betrieb der Feuerungsanlage unterbrochen werden müsste.

Im Vergleich zu der vorbekannten Sonde wird die Messkammer durch das Verbindungselement mit dem kleineren Innendurchmesser in axialer Richtung nach hinten verlagert. Die Messkammer kann weiterhin innerhalb des Sondenrohrs angeordnet sein. Sie kann aber auch nahe dem kalten Ende des Sondenrohrs, welches sich außerhalb des Feuerraums befindet, in einem angeflanschten Gehäuse angeordnet sein.

Es sei angemerkt, dass die Merkmale der Erfindung sich vorzugsweise aber nicht ausschließlich auf kreisrunde Elemente mit konstantem Durchmesserwert in allen Richtungen beziehen. Sowohl der Durchmesser des Innenmantels als auch der Durchmesser des Verbindungselements können variieren. Innenmantel und Verbindungselement können z.B. einen viereckigen oder dreieckigen Querschnitt aufweisen. Der Ringraum muss nicht einen Kreisring mit homogener Erstreckung entlang des gesamten Umfangs sein. Es kann auch ein eckiger Ring oder in sonstiger Weise inhomogener Ring sein. Der Ringraum hat aber eine größere Querschnittsfläche als der Ringspalt zwischen Ausstoßplatte und Innenmantel, so dass sich in dem Ringraum die Messgasströmung erheblich verlangsamt. Die radiale Erstreckung des Ringraums muss nicht an allen Stellen einem Mehrfachen der radialen Erstreckung des Ringspalts entsprechen. Insbesondere bei von der Kreisringform abweichenden Querschnitten des Ringraums ist es ausreichend dass die mittlere radiale Erstreckung des Ringraums einem Mehrfachen der mittleren radialen Erstreckung des Ringspalts entspricht, so dass sich insgesamt eine erheblich viel größere Querschnittsfläche des Ringraums im Vergleich zum Ringspalt ergibt.

In der Praxis haben sich folgende Merkmale als vorteilhaft erwiesen, welche einzeln oder in Kombination miteinander die oben beschriebene Gasentnahmesonde vorteilhaft weiterbilden.

Die Länge des Ringraums kann größer sein als der Außendurchmesser des Sondenrohrs. Je länger der Ringraum ist, desto länger strömt das mit Partikeln beladene Gas mit verlangsamter Geschwindigkeit, bevor es den Filter erreicht, und desto mehr Partikel können sich in dem Ringraum ablagern, so dass sie nicht die Filteroberfläche verschmutzen.

Das Verbindungselement kann sich über mindestens ein Drittel der Länge des Sondenrohrs erstrecken. Bei einem 2 m langen Sondenrohr wäre die Länge des Verbindungselements folglich mindestens 60 cm. In der Praxis kann die Länge des Verbindungselements sogar größer als die Hälfte der Länge des Sondenrohrs sein.

Der Filter kann sich über mindestens ein Viertel der Länge des Sondenrohrs erstrecken. Bei einem 2 m langen Sondenrohr hätte der Filter folglich eine Mindestlänge von 50 cm. Es können auch mehrere Filter hintereinander angeordnet sein.

Die Messkammer grenzt in axialer Richtung an das vom Verbindungselement abgewandte Ende des Filters. Insbesondere sind dann innerhalb des Innenmantels des Sondenrohrs ausgehend von dessen freien Ende folgende beweglichen Elemente in axialer Richtung aufeinanderfolgend angeordnet:
- Ausstoßplatte
- Verbindungselement
- Filter (einer oder mehrere).

Die Messkammer schließt sich in einer praktischen Ausführungsform unmittelbar an das kalte Ende des Sondenrohrs an, kann aber auch innerhalb des Sondenrohrs im Bereich des kalten Endes angeordnet sein. Die Messkammer ist fest mit den beweglichen Elementen in dem Sondenrohr gekoppelt.

Diese beweglichen Elemente sind in der Praxis in axialer Richtung verschiebbar in dem Innenmantel des Sondenrohrs aufgenommen und mit einer Antriebseinheit verbunden. Die Antriebseinheit drückt in regelmäßigen Abständen die Elemente in axialer Richtung nach vorne, so dass die Ausstoßplatte durch das freie Ende des Sondenrohrs austritt. Gleichzeitig wird Spülgas innerhalb des Innenmantels über den Filter und an dem Verbindungselement vorbei aus dem freien Ende des Sondenrohrs heraus geleitet. Das Spülgas ist zu diesem Zweck in einem Spülgastank mit erheblichem Überdruck aufgenommen. Das Spülgas entfernt Ablagerungen von der äußeren Oberfläche des Filters und spült diese durch den Ringraum zwischen Verbindungselement und Innenmantel des Sondenrohrs zum freien Ende des Sondenrohrs und dann zurück in das Rauchgas. Ablagerungen an der Stirnseite des Sondenrohrs werden durch die Ausstoßplatte entfernt. Aufgrund des großen Volumenstroms an Spülgas wird vermieden, dass derartige Ablagerungen in den Ringraum zwischen Verbindungselement und Innenmantel geraten. Die am freien Ende des Sondenrohrs austreten Spülgasströmung vermeidet ein Eindringen von Partikeln aus der Rauchgasströmung in das Sondenrohr.

Wie bereits weiter oben erwähnt, kann sich die Messkammer innerhalb des Sondenrohrs befinden. Insbesondere kann sich die Messkammer nahe dem kalten Ende des Sondenrohrs befinden, welches dem freien Ende des Sondenrohrs gegenüberliegt, das in die Rauchgasströmung hineinragt.

In der Praxis kann die Länge des Sondenrohrs zwischen 2.000 und 4.000 mm (2 m und 4 m) betragen.

Der Durchmesser des Sondenrohrs kann insbesondere zwischen 80 und 200 mm liegen.

Die Breite des Ringspalts kann in der Praxis kleiner als 1 mm sein. Insbesondere hat sich ein Ringspalt mit einer Breite von etwa 0,5 mm bewährt.

Die Breite des Ringraums zwischen dem Verbindungselement und dem Innenmantel des Sondenrohrs kann größer als 5 mm sein. Auf diese Weise wird die Geschwindigkeit der Strömung des Messgases zum Filter hin innerhalb des Innenmantels des Sondenrohrs erheblich reduziert und die Ablagerung von in dem Messgas befindlichen Partikeln ermöglicht.

In der Praxis kann der mindestens eine rohrförmige Filter von einem Druckrohr durchragt sein, welches mindestens eine Durchlassöffnung aufweist und dessen Innenraum mit der Messkammer in Fluidverbindung steht. Folglich kann das Messgas nicht nur durch den Ringraum zwischen Filter und Druckrohr zur Messkammer strömen, sondern auch durch den Innenquerschnitt des Druckrohrs.

In der Praxis kann ferner die Ausstoßplatte mit Zentrierkörpern verbunden sein, die sich gegen die Wand des Innenmantels abstützen. Insbesondere kann die Ausstoßplatte an einem Führungselement befestigt sein, welches sich in radialer Richtung erstreckende Stifte aufweist. Die Stifte liegen gegen den Innenmantel des Sondenrohrs an. Der Innenmantel des Sondenrohrs besteht üblicherweise aus Edelstahl. Die Stifte können aus einem weicheren Metall, beispielsweise Messing bestehen. Auf diese Weise wird die Ausstoßplatte präzise unter Einhaltung des Ringspaltes in dem Sondenrohr geführt. Vorzugsweise sind 3 Messingstifte als Zentrierkörper in dem Führungselement angeordnet. Die drei Messingstifte erstrecken sich in radialer Richtung und sind jeweils um einen Winkel von 120° zueinander versetzt. Es können aber auch mehr Zentrierkörper vorgesehen sein.

Die Ausstoßplatte und das Führungselement mit den Zentrierkörpern können lösbar mit den weiteren beweglichen Elementen im Sondenrohr (Verbindungselement, Filter) verbunden sein. Die Außenseite der Ausstoßplatte befindet sich in unmittelbarem Kontakt mit dem Rauchgas. Die Messingstifte, welche die Führungselemente für die Ausstoßplatte bilden, können verschleißen, weil sie aus einem weicheren Metall als der Innenmantel bestehen. Durch die Möglichkeit des Austausches der stark belasteten Elemente und der verschleißenden Elemente lässt sich die erfindungsgemäße Sonde über einen sehr langen Zeitraum sehr zuverlässig betreiben.

Die Erfindung betrifft ferner ein Verfahren zur Messung der Zusammensetzung partikelbeladener Gase, bei dem
- Messgas mittels eines von Kühlflüssigkeit durchströmten Sondenrohrs mit einem Außenmantel und einem Innenmantel aus einer Gasströmung entnommen wird,
- das Messgas durch einen schmalen Ringspalt zwischen einer in dem Innenmantel angeordneten Ausstoßplatte und dem Innenmantel im Bereich des freien Endes des Sondenrohrs in das Sondenrohr einströmt, und
- das Messgas durch einen rohrförmigen Filter aus Sinterwerkstoff zu einer Messkammer mit einem Messsensor geleitet wird.

Wiederum mit dem Ziel, ein Zusetzen des Filters zu vermeiden, wird das Messgas vor dem Einströmen in den Filter durch einen zwischen dem Filter und der Ausstoßplatte angeordneten Ringraum geleitet, dessen radiale Erstreckung dem Mehrfachen der radialen Erstreckung des Ringspalts zwischen Ausstoßplatte und Innenmantel entspricht.

In der Praxis kann die Ausstoßplatte in vorgegebenen Zeitintervallen axial durch das freie Ende aus dem Sondenrohr herausbewegt werden, wobei ein Spülgas mit Überdruck in den Innenmantel geleitet wird und über die Filteroberfläche und durch den Ringraum hindurch und aus dem freien Ende des Sondenrohrs heraus strömt. Aufgrund des vergrößerten Strömungsquerschnitts in dem Ringraum ist der Volumenstrom des Spülgases bei der Sonde erheblich viel größer als bei der vorbekannten Sonde, bei der zwischen dem Innenmantel und den darin beweglichen Teilen der Sonde nur ein sehr kleiner Ringspalt existierte. Durch die vergrößerte Spülgasmenge wird ein wesentlich höheres Strömungsvolumen erreicht, das jegliche Ablagerung von der Filteroberfläche entfernt. Ferner werden sämtliche Ablagerungen zuverlässig aus dem Ringraum heraus geblasen. Das Spülgas wird mit einem erheblichen Überdruck in einem großen Tank (z.B. 30 L) bevorratet, so dass zu Beginn des Spülvorgangs ein Druckstoß in dem Innenmantel entsteht. Dieser Druckstoß reißt Ablagerungen auf der Filteroberfläche und an anderen Oberflächen innerhalb des Innenmantels mit. Es ist ausreichend Spülgas vorhanden, um eine Strömung über einen andauernden Zeitraums von mehreren Sekunden aufrecht zu erhalten, bis die Ausstoßplatte wieder in das Sondenrohr hineinbewegt wird und die Gaseintrittsöffnung bis auf den Ringspalt verschließt.

In der Praxis ist das Sondenrohr mit einer Antriebsvorrichtung verbunden, welche das Sondenrohr in vorgegebenen Zeitintervallen in axialer Richtung bewegt. Diese Bewegung erfolgt über einen Bruchteil der Länge des Sondenrohrs. Ein 2 - 4 m langes Sondenrohr kann zum Beispiel um 0,5 m oder 1 m axial aus dem Feuerraum herausgezogen und dann wieder eingeschoben werden. Durch die axiale Verschiebung wird sichergestellt, dass sich keine unzulässig großen Ablagerungen auf dem Sondenrohr in der Nähe der Eintrittsöffnung in der Wand des Feuerraums bilden. Durch das Herausziehen in axialer Richtung werden auf dem Sondenrohr in der Nähe der Wand des Feuerraums entstandene Ablagerungen durch den Rand der Eintrittsöffnung für das Sondenrohr abgestreift.

Darüber hinaus kann das Sondenrohr wie bei der vorbekannten Ausführungsform gedreht werden. Durch das Drehen wird sichergestellt, dass aufeinanderfolgend unterschiedliche Oberflächenbereiche des Sondenrohrs der Strömung ausgesetzt sind, so dass sich die Ablagerungen auf verschiedene Bereiche des Sondenrohrs verteilen und lose Partikel von der Oberfläche des Sondenrohrs herunter fallen.

Weitere praktische Ausführungsformen und Vorteile der Erfindung sind nachfolgend im Zusammenhang mit den Zeichnungen beschrieben.
Figur 1 zeigt eine dreidimensionale Ansicht einer erfindungsgemäßen Gasentnahmesonde.
Figur 2 zeigt eine geschnittene Ansicht des Sondenrohrs der Gasentnahmesonde aus Figur 1.
Figur 3 zeigt eine vergrößerte Darstellung des Details A aus Figur 2.
Figur 4 zeigt eine vergrößerte Darstellung des Details B aus Figur 2.
Figur 5 zeigt eine geschnittene Seitenansicht der aufeinander folgenden beweglichen Elemente innerhalb des Sondenrohrs.
Figur 6 zeigt eine vergrößerte Darstellung des Details C aus Figur 5.
Figur 7 zeigt eine vergrößerte Darstellung des Details D aus Figur 5.
Figur 8 zeigt eine vergrößerte Darstellung des Details E aus Figur 5.
Figur 9 zeigt eine vergrößerte Darstellung des Details F aus Figur 5.
Figur 10 zeigt eine Seitenansicht der beweglichen Teile aus Figur 5.
Figur 11 zeigt eine vergrößerte Schnittansicht entlang Schnittlinie XI-XI in Figur 10.
Figur 12 zeigt eine dreidimensionale Ansicht des Sondenrohrs mit daran anschließender Messkammer in teilgeschnittener Darstellung.
Figur 13 zeigt eine vergrößerte Darstellung des Details G aus Figur 12.

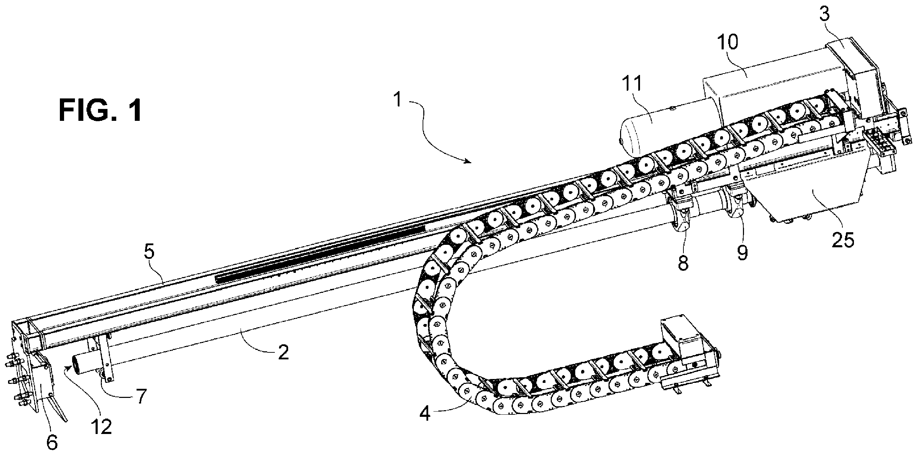

In Figur 1 ist in dreidimensionaler Draufsicht eine durch eine Kühlflüssigkeit gekühlte Gasentnahmesonde 1 gemäß der hier beschriebenen Entwicklung dargestellt. Als Kühlflüssigkeit wird in der Regel Waser verwendet. Die Gasentnahmesonde 1 besteht im Wesentlichen aus einem Sondenrohr 2 mit einem Anschlusskasten 3, der am hinteren Ende des Sondenrohrs 2 befestigt ist. Der Anschlusskasten 3 ist über eine Energiezuführungskette 4 mit ortsfesten Vorrichtungen (nicht dargestellt) wie einer Pumpe und einem Wärmetauscher für das Kühlwasser, einem Analyseschrank, einer Zufuhr für Spülgas, einer Stromversorgung usw. verbunden. Die Energiezuführungskette 4 besteht aus mehreren Gliedern und nimmt sämtliche Kabel und Schläuche auf, über die der Anschlusskasten 3 an stationäre Einrichtungen angeschlossen ist. Der Anschlusskasten 3 umfasst hauptsächlich Ventile für pneumatische und hydraulische Leitungen, da die Antriebe der Gasentnahmesonde vorzugsweise pneumatisch betätigt werden. Die Glieder der Energiezuführungskette 4 ermöglichen eine beschädigungsfreie Bewegung der Kabel und Schläuche während des Verschiebens des Sondenrohrs 2. Das Sondenrohr 2 hat im vorliegenden Fall eine Länge von 4 m und einen Durchmesser von 120 mm. Das Sondenrohr 2 ist an einer Tragschiene 5 in axialer Richtung verschiebbar befestigt. Die Tragschiene 5 besteht aus zwei I-förmigen Stahlträgern, die an ihren Enden durch geeignete Verbindungselemente miteinander verbunden sind. Die Tragschiene 5 kann Zahnstangen aufweisen, welche mit Zahnrädern eines Antriebsmotors 10 für die Gasentnahmesonde zusammenwirkt. An der Tragschiene 5 können ferner Bewegungsanschläge und Bewegungsmelder für die Steuerung der Bewegung der Gasentnahmesonde 1 vorgesehen sein. Am vorderen Ende der Tragschiene 5 ist ein Verschlusskasten 6 angeordnet. Dieser wird an einer Wandung des rauchgasführenden Raumes, beispielsweise eines Feuerraumes eines Zementdrehrohrofens angeflanscht. Der Verschlusskasten 6 sorgt dafür, dass sowohl bei eingeschobenem Sondenrohr 2 als auch bei ausgezogenem Sondenrohr 2, wie in Figur 1 dargestellt, die Öffnung zum Feuerraum bestmöglich verschlossen ist.

Im vorderen Bereich der Tragschiene 5 ist eine Tragrolle 7 befestigt, welche das Sondenrohr 2 abstützt. Der hintere Bereich des Sondenrohrs 2 ist über Stehlager 8, 9 mit einem Verfahrschlitten verbunden, der auf den zwei Trägern der Tragschiene 5 verfährt. Die Stehlager 8,9 sind Teil der verfahrbaren Einheit. Mit dem Anschlusskasten 3 ist der Antriebsmotor 10 verbunden. Der Antriebsmotor ist ein Pneumatikmotor und verschiebt die Einheit bestehend aus dem Verfahrschlitten mit Sondenrohr 2 mit Anschlusskasten 3 und Antriebsmotor 10 in axialer Richtung entlang der Tragschiene 5. Auf dem Verfahrschlitten ist ein Gasbehälter 11 befestigt. Dieser dient der Aufnahme von Spülgas, welches während des weiter unten beschriebenen Reinigungsvorgangs der Gasentnahmesonde 1 verwendet wird.

Zum Messbetrieb wird das Sondenrohr 2 durch den Antriebsmotor 10 in axialer Richtung in Richtung seines freien Endes 12 verschoben. Dabei tritt das freie Ende 12 des Sondenrohrs 2 durch den Verschlusskasten 6 in den Feuerraum ein und wird um mehr als einen Meter in die Rauchgasströmung hineinbewegt.

Das Rauchgas in dem Feuerraum hat beispielsweise im Fall eines Zementdrehrohrofens eine Temperatur von bis zu 1.400°C. Aus diesem Grund ist das Sondenrohr 2 gekühlt, im vorliegenden Fall wassergekühlt.

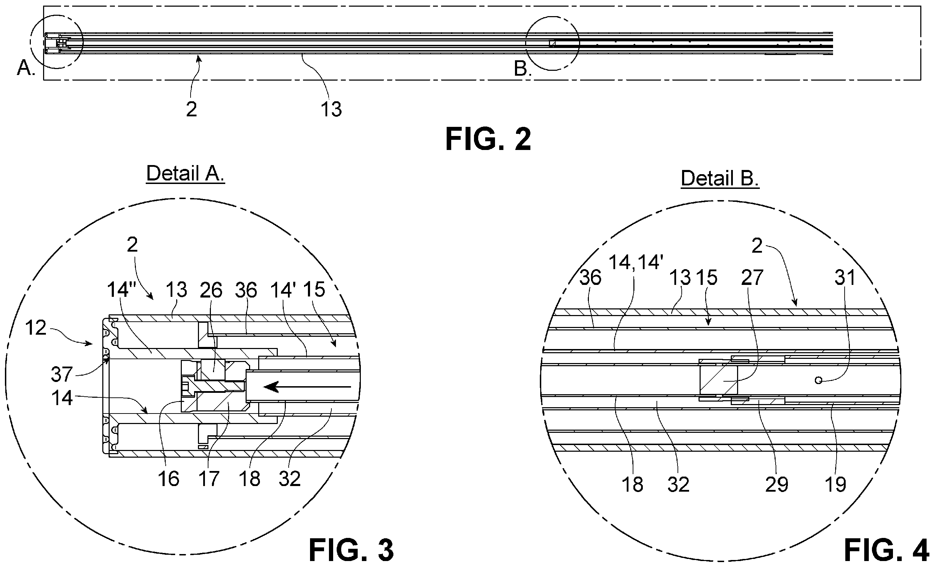

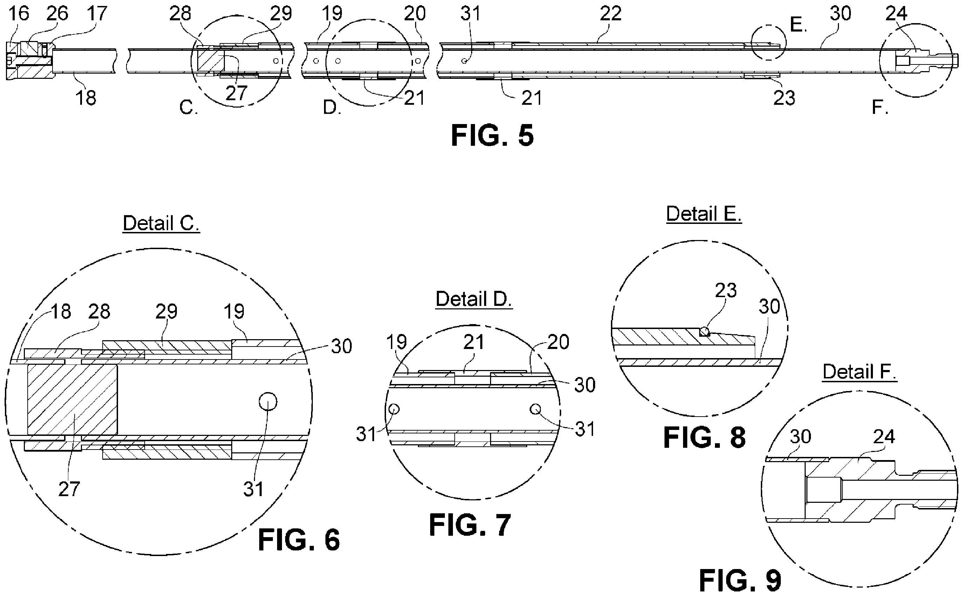

Die Figuren 2 bis 11 erläutern den Aufbau des Sondenrohrs 2 und der darin beweglichen Elemente, welche wie in Fig. 5 erkennbar eine bewegliche Einheit bilden, die auch Plunger genannt wird. Wie in den Figuren 2 bis 4 zu erkennen ist, besteht das Sondenrohr 2 aus einem Außenmantel 13, einem Zwischenmantel 36 und einem Innenmantel 14. Der Innenmantel 14 setzt sich zusammen aus einem inneren Mantelrohr 14' und einem hülsenförmigen Abschnitt 14" eines Endstücks 37, das den Außenmantel 13 mit dem Innenmantel 14 im Bereich des freien Endes 12 des Sondenrohrs 2 dicht verbindet. Zwischen Außenmantel 13 und Innenmantel 14 wird ein Strömungsraum 15 für die Kühlflüssigkeit gebildet. Der Strömungsraum 15 ist durch den Zwischenmantel 36 in zwei längliche Räume mit ringförmigem Querschnitt unterteilt. Im äußeren Zuströmraum wird relativ kaltes Wasser von dem kalten Ende des Sondenrohrs 2 zu dem freien Ende 12 des Sondenrohrs 2 in dem Rauchgas geleitet. Im inneren Abströmraum strömt das Wasser zurück zum kalten Ende des Sondenrohrs 2. In dem Strömungsraum 15 können weitere Führungseinrichtungen, insbesondere Führungsbleche (nicht dargestellt) vorgesehen sein, welche die Wasserströmung innerhalb des Strömungsraums 15 beeinflussen. Wassereintritt und Wasseraustritt sind in bekannter Weise beide am Ende des kalten Ende Sondenrohrs nahe dem Anschlusskasten 3 (s. Fig. 1) angeordnet. Die zirkulierende Wasserströmung innerhalb des Strömungsraums 15 sorgt für eine effektive Kühlung des Sondenrohrs 2 und der darin befindlichen Elemente.

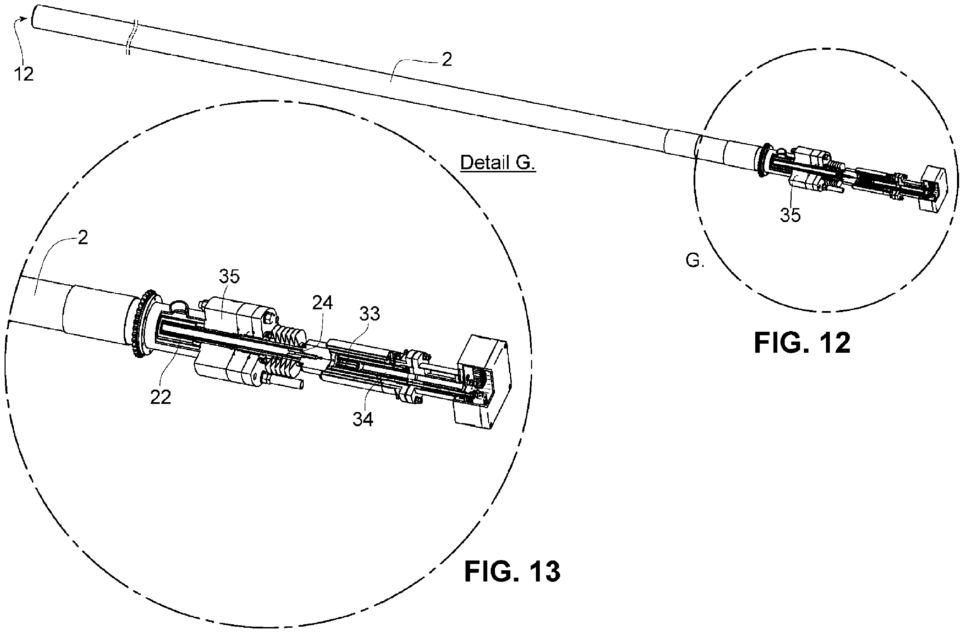

Die in axialer Richtung aufeinander folgenden Elemente innerhalb des Innenmantels 14 des Sondenrohrs 2 sind insbesondere in den Figuren 2 bis 13 zu erkennen. Sie umfassen
- eine Ausstoßplatte 16, welche am nächsten zum freien Ende 12 des Sondenrohrs 2 positioniert ist;
- ein Führungselement 17, an dem die Ausstoßplatte 16 befestigt ist;
- ein rohrförmiges Verbindungselement oder Verbindungsrohr 18;
- einen ersten rohrförmigen Filter 19 aus Sintermetall;
- einen zweiten rohrförmigen Filter 20 aus Sintermetall, der mit dem ersten Filter 19 über eine Verbindungsmuffe 21 verbunden ist;
- ein Verlängerungselement 22, welches über einen Dichtring 23 an seinem von der Ausstoßplatte 16 entfernten Ende gegenüber einer Messkammer 33; und
- ein Anschlussstück 24, über welches die beweglichen Elemente mit der Messkammer 33 verbunden werden.
   Die Messkammer 33 befindet sich hinter dem kalten Ende des Sondenrohrs 2 hinter einer Verkleidung 25 (siehe Figuren 1 und 13). In der Messkammer 33 sind aus dem Stand der Technik bekannte Sensoren angeordnet, insbesondere ein Sauerstoffsensor sowie ein Sensor zum Messen des Kohlenmonoxidgehalts (CO).

Im Gegensatz zum Stand der Technik ist die Messkammer bei der vorliegenden Gasentnahmesonde nahe dem kalten Ende des Sondenrohrs 2 angeordnet, welches dem freien Ende 12 gegenüberliegt. Die Sensoren befinden sich somit nicht innerhalb der Rauchgasströmung sondern in einem relativ kühlen Bereich. Hierdurch werden die Zuverlässigkeit und die Lebensdauer der Sensoren erheblich erhöht, wobei ihre Nähe zum Ort der Gasentnahme, d.h. zum freien Ende 12 des Sondenrohrs 2 im Rauchgas nach wie vor gewährleistet ist.

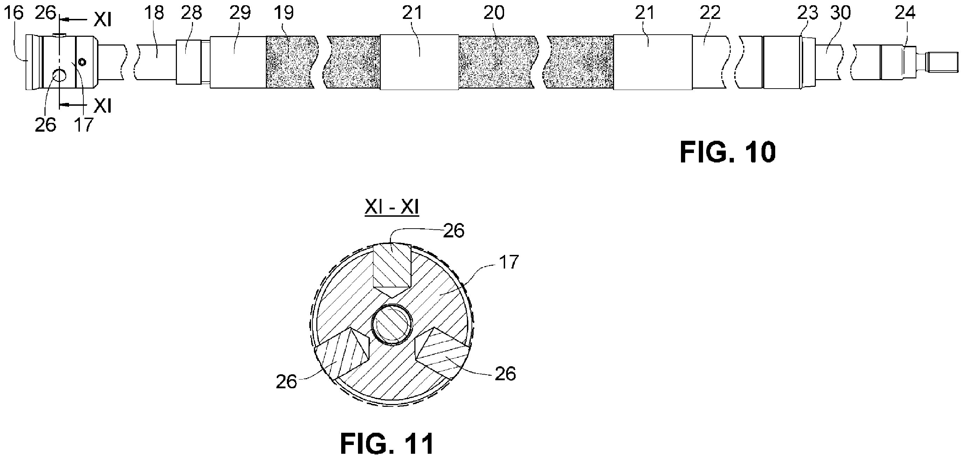

Das Detail A in Figur 3 zeigt, wie die Ausstoßplatte 16 an dem Führungselement 17 und dieses an dem Verbindungsrohr 18 befestigt ist. Das Verbindungsrohr 18 ist an das von dem freien Ende 12 des Sondenrohrs 2 abgewandte Ende des Führungselements 17 geschweißt. Auf der gegenüberliegenden Seite ist die Ausstoßplatte 16 mit einer Schraubverbindung an dem Führungselement 17 befestigt. Die Ausstoßplatte 16 steht in direktem Kontakt mit dem Rauchgas und wird in regelmäßigen Zeitabständen in der in Figur 3 durch den Pfeil dargestellten Bewegungsrichtung aus dem Sondenrohr 2 heraus geschoben, bis sie um mehrere Millimeter aus dem Sondenrohr 2 heraus ragt. Die Ausstoßplatte 16 ist daher für Verschleiß anfällig und mittels der Verschraubung ohne großen Aufwand von dem Führungselement 17 lösbar. Das Führungselement 17 weist drei Zentrierkörper 26 auf (siehe Figuren 10 und 11). Die Zentrierkörper 26 bestehen aus zylindrischen Messingstiften. Sie sind in radiale Bohrungen im Führungselement 17 eingesteckt, deren Bohrungsachse jeweils um 120° zur nächsten Bohrung versetzt ist. Die Oberflächen der Zentrierkörper 26 aus Messing liegen gegen den Innenmantel 14 des Sondenrohrs 2 aus Stahl an (siehe Figur 3). Beim Zusammenbau der Sonde wird das Führungselement 17 mit der Ausstoßplatte 16 innerhalb des Innenmantels 14 durch die gesamte Länge des inneren Mantelrohrs 14' bis in den hülsenförmigen Abschnitt 14" des Endstücks 37 geschoben. Während des Betriebs wird das Führungselement 17 mit der Ausstoßplatte 16 innerhalb des hülsenförmigen Abschnitt 14" des Endstücks 37 und daraus heraus bewegt. Die Zentrierkörper 26 gewährleisten, dass zwischen dem Umfang der Ausstoßplatte 16 und dem Innenmantel 14 ein schmaler ringförmiger Spalt eingehalten wird. Der ringförmige Spalt ist relativ zum Durchmesser der Ausstoßplatte 16 so klein, dass er in Figur 3 kaum zu erkennen ist. Die Spaltbreite kann z.B. in der Größenordnung von 0,5 mm liegen. Da das Material der Zentrierkörper 26 (Messing) weicher als das Material des Innenmantels 14 (Stahl) ist, wird der Innenmantel 14 bei der Relativbewegung des Führungselements 17 zum Sondenrohr 2 nicht verschlissen. Die Zentrierkörper 26 können dagegen relativ einfach ausgetauscht werden.

Es ist beispielsweise in Figur 3 oder Figur 5 zu erkennen, dass die in dem Innenmantel 14 befindliche bewegliche Anordnung kurz hinter der Ausstoßplatte 16 einen reduzierten Durchmesser aufweist. Bereits das Führungselement 17 hat einen geringeren Durchmesser als die Ausstoßplatte 16. Das sich an das Führungselement 17 anschließende Verbindungselement (Verbindungsrohr 18) hat einen um mehrere Millimeter kleineren Durchmesser als die Ausstoßplatte 16. Der Durchmesser der Ausstoßplatte liegt in der Größenordnung von über 40 mm. Der Durchmesser des Verbindungsrohrs 18 liegt bei weniger als 30 mm.

Das Verbindungsrohr 18 weist an seinem von der Ausstoßplatte 16 abgewandten Ende einen Verschlusskörper 27 und eine Schraubmuffe 28 auf. Die Schraubmuffe 28 kann mit einer komplementären Schraubmuffe 29 verbunden werden, welche an den ersten rohrförmigen Filter 19 aus Sintermetall angeschweißt ist. Der erste rohrförmige Filter 19 hat eine Länge von etwa 0,5 m. Es schließt sich ein zweiter rohrförmiger Filter 20 an, der eine Länge von 1 m hat. Die Gesamtlänge der rohrförmigen Filter 19, 20 ermöglicht den Durchtritt eines hinreichend großen Volumenstroms an Messgas während der Messung.

Die beiden rohrförmigen Filter 19, 20 sind durch eine Verbindungsmuffe 21 miteinander verbunden, welche insbesondere in Figur 7 dargestellt ist. Über eine entsprechende Verbindungsmuffe 21 ist der Filter 20 mit einem rohrförmigen Verlängerungselement 22 aus Edelstahl verbunden. Das Ende des rohrförmigen Verlängerungselements 22 ist mit einem Bauelement verbunden, in dem sich die Messkammer befindet, und über einen Dichtring 23 abgedichtet.

Innerhalb der Filter 19, 20 und innerhalb des rohrförmigen Verlängerungselements 22 erstreckt sich ein Druckrohr 30. Das Druckrohr 30 ist an seinem vorderen Ende mit dem Verschlusskörper 27 dicht verschlossen und in axialer Richtung mit dem rohrförmigen Verbindungselement 18 gekoppelt (siehe Fig. 6). Am gegenüberliegenden Ende weist das Druckrohr 30 das Anschlussstück 24 auf, welches mit der Messkammer 33 verbindbar ist. Das Druckrohr 30 weist insbesondere im Bereich des rohrförmigen Filters 19, 20 Durchlassöffnungen 31 auf, welche das Hindurchströmen von Gas durch die Wandung des Druckrohrs 30 ermöglichen. Auf diese Weise kann Messgas nicht nur durch den ringförmigen Raum zwischen dem Druckrohr 30 und dem Verlängerungselement 22 bzw. den Filtern 19, 20 hindurchströmen sondern insbesondere durch den Innenraum des Druckrohrs 30.

Während des Messbetriebes befindet sich die Ausstoßplatte mit den sich daran anschließenden axial verschiebbaren Elementen vollständig innerhalb des Sondenrohrs 2 gemäß der in Figur 2, 3 und 4 gezeigten Darstellung. Insbesondere in Figur 3 ist zu erkennen, dass zwischen der Ausstoßplatte 16 und dem vorderen Ende des Innenmantels 14 ein sehr schmaler Spalt verbleibt. Der Spalt wird präzise aufgrund der Führung des der Ausstoßplatte 16 mittels der Zentrierkörper 26 in dem Innenmantel 14 des Sondenrohrs 2 eingestellt. Der Bereich der Ausstoßplatte 16 mit dem größten Durchmesser erstreckt sich nur über wenige Millimeter. Danach reduziert sich der Durchmesser der Ausstoßplatte 16 und des anschließenden Führungselements 17. Das Führungselement 17 selbst ist an dem rohrförmigen Verbindungselement 18, auch Verbindungsrohr genannt, befestigt, dessen Durchmesser mehrere Millimeter kleiner als der Durchmesser des Innenmantels 14 ist.

In der Messkammer 33 herrscht im Messbetrieb ein Unterdruck, hervorgerufen durch eine saugende extern anzuschließende Pumpe, so dass aus der Rauchgasströmung durch die Öffnung in dem freien Ende 12 des Sondenrohrs 2 eine bestimmte Menge an Messgas entnommen wird. Zunächst strömt das Messgas durch den ringförmigen schmalen Spalt zwischen Ausstoßplatte 16 und Innenmantel 14. Dann erweitert sich der Spalt und geht in den Ringraum 32 über, der sich zwischen dem Verbindungsrohr 18 und dem Innenmantel 14 befindet. In diesem Ringraum 32 ist die Strömungsgeschwindigkeit sehr viel kleiner als in dem Spalt zwischen Ausstoßplatte 16 und dem Innenmantel 14. Partikel, die durch den genannten Spalt hindurchgetreten sind, setzen sich aufgrund der langsamen Strömungsgeschwindigkeit in dem Ringraum 32 ab. Der Ringraum 32 erstreckt sich über eine längere Strecke, z.B. 0,6 bis 1 m. Die meisten Partikel werden aus dem Messgas beim Durchströmen des Ringraums 32 aufgrund der Schwerkraft absinken und sich nicht bis zur Oberfläche der Filter 19 oder 20 bewegen. Durch spiralförmige Nuten in der Außenfläche der Ausstoßplatte 16 oder an der Innenwand des Innenmantels 14 kann der Messgasströmung auch eine Drehbewegung aufgeprägt werden, welche einen Vortex-Effekt erzeugt und durch Zentrifugalkraft zusätzlich Partikel aus der Messgasströmung ausscheidet.

An der Oberfläche der rohrförmigen Filter 19 und 20 weist das Messgas kaum noch Partikel auf. Das Messgas strömt durch die rohrförmigen Filter 19, 20 von deren Außenseite zu deren Innenseite. Hier kann das Messgas entweder außerhalb des Druckrohrs 30 oder durch die Durchlassöffnungen 31 in dem Druckrohr 30 hindurch und innerhalb des Druckrohrs 30 in axialer Richtung weiter zur Messkammer strömen. An den zweiten Filter 20 aus Sintermaterial schließt sich ein Verbindungselement 22 aus Edelstahlrohr an, dessen Ende mittels des Dichtrings 23 abgedichtet ist und zur Messkammer führt. Auch das Ende des Druckrohrs 30 ist über das Anschlussstück 24 mit der Messkammer 33 verbunden (s. Fig. 13). Das entnommene Messgas strömt folglich zum einen zwischen dem Druckrohr 30 und dem rohrförmigen Verlängerungselement 22 und zum anderen innerhalb des Druckrohrs 30 zur Messkammer 33. In der Messkammer befindet sich die eigentliche Messsonde 34 für die Gasanalyse mit den hierfür erforderlichen Sensoren.

Zum Reinigen der hier beschriebenen Vorrichtung wird mittels eines Antriebs das Druckrohr 30, die Filter 19, 20 und das Verbindungsrohr 18 nach vorne zum freien Ende 12 des Sondenrohrs hin gedrückt. Dadurch bewegt sich das Führungselement 17 mit der Ausstoßplatte 16 in der in Figur 3 durch den Pfeil dargestellten Richtung aus dem freien Ende 12 des Sondenrohrs 2 heraus. Gleichzeitig wird dem Innenmantel 14 aus dem Gasbehälter 11 Spülgas mit einem hohen Druck zugeführt. Das Spülgas strömt zunächst mit einem Druckstoß und dann kontinuierlich durch den Innenmantel 14 entlang den Oberflächen der Filter 19, 20. Dabei reißt das Spülgas eventuelle Verschmutzungen, die sich an den Filtern 19, 20 abgelagert haben, mit. Weiter strömt das Spülgas parallel zu dem Verbindungsrohr 18 in Richtung des freien Endes 12 des Sondenrohrs 2 und hier aus dem Sondenrohr 2 heraus. Sämtliche Verunreinigungen und Partikel innerhalb des Sondenrohrs 2 werden dadurch aus dem Sondenrohr 2 ausgeblasen. Noch während der Strömung des Spülgases werden die beweglichen Elemente innerhalb des Sondenrohrs 2 wieder zurückgezogen, so dass sich die Ausstoßplatte 16 in das Sondenrohr 2 in die in Figur 3 erkennbare Stellung zurückbewegt. Anschließend wird die Spülgaszufuhr zum Sondenrohr unterbrochen und die Messkammer wird mit einem Unterdruck in Bezug auf das Rauchgas beaufschlagt, so dass wiederum Rauchgas durch das Sondenrohr 2 und die darin angeordneten Filter 19, 20 zur Messkammer 33 am kalten Ende des Sondenrohrs 2 strömt.

Die in der vorliegenden Beschreibung, in den Zeichnungen sowie in den Ansprüchen offenbarten Merkmale der Erfindung können sowohl einzeln als auch in beliebigen Kombinationen für die Verwirklichung der Erfindung in ihren verschiedenen Ausführungsformen wesentlich sein. Die Erfindung ist nicht auf die beschriebenen Ausführungsformen beschränkt. Sie kann im Rahmen der Ansprüche und unter Berücksichtigung der Kenntnisse des zuständigen Fachmanns variiert werden.

### Bezugszeichen liste

- 1: Gasentnahmesonde
- 2: Sondenrohr
- 3: Anschlusskasten
- 4: Energiezuführungskette
- 5: Tragschiene
- 6: Verschlusskasten
- 7: Tragrolle
- 8: Stehlager
- 9: Stehlager
- 10: Antriebsmotor
- 11: Gasbehälter
- 12: freies Ende
- 13: Außenmantel
- 14: Innenmantel
- 15: Strömungsraum
- 16: Ausstoßplatte
- 17: Führungselement
- 18: Verbindungsrohr, rohrförmiges Verbindungselement
- 19: rohrförmiger Filter
- 20: rohrförmiger Filter
- 21: Verbindungsmuffe
- 22: Verlängerungselement
- 23: Dichtring
- 24: Anschlussstück
- 25: Verkleidung
- 26: Zentrierkörper
- 27: Verschlusskörper
- 28: Schraubmuffe
- 29: Schraubmuffe
- 30: Druckrohr
- 31: Durchlassöffnung
- 32: Ringraum
- 33: Messkammer
- 34: Messsonde
- 35: Dichtungselement
- 36: Zwischenmantel

## Patentansprüche

1. Flüssigkeitsgekühlte Gasentnahmesonde (1) mit:
- einem von Kühlflüssigkeit durchströmten Sondenrohr (2) mit einem Außenmantel (13) und einem Innenmantel (14), die dicht miteinander verbunden sind und die Begrenzung eines Strömungsraums (15) bilden,
- mindestens einem rohrförmigen Filter (19,20) aus Sinterwerkstoff, der innerhalb des Innenmantels (14) des Sondenrohrs (2) angeordnet ist, wobei durch das freie Ende (12) des Sondenrohrs (2) in den Innenmantel (14) des Sondenrohrs (2) eintretendes Messgas den rohrförmigen Filter (19,20) von außen nach innen durchströmt,
- mindestens einer Messkammer (33) mit mindestens einem Messsensor, in die das Messgas nach dem Durchströmen des rohrförmigen Filters (19,20) eintritt,
- einer Ausstoßplatte (16), welche im Bereich des freien Endes (12) des Sondenrohrs (2) angeordnet und innerhalb des Innenmantels (14) axial verschiebbar ist, wobei im Messbetrieb ein schmaler Ringspalt zwischen der Außenseite der Ausstoßplatte (16) und dem Innenmantel (14) des Sondenrohrs (2) das Einströmen von Messgas in das Sondenrohr (2) ermöglicht,
**dadurch gekennzeichnet, dass** zwischen dem Filter (19,20) und der Ausstoßplatte (16) ein Verbindungselement (18) angeordnet ist, dessen Durchmesser mehrere Millimeter kleiner ist als der Innendurchmesser des Innenmantels (14), so dass zwischen dem Verbindungselement (18) und dem Innenmantel (14) ein Ringraum (32) entsteht, dessen radiale Erstreckung einem Mehrfachen der radialen Erstreckung des Ringspalts zwischen Ausstoßplatte (16) und Innenmantel (14) entspricht.

2. Gasentnahmesonde (1) nach Anspruch 1, **dadurch gekennzeichnet, dass** sie mindestens eines der folgenden Merkmale aufweist:
- die Länge des Verbindungselements (18) ist größer als der Außendurchmesser des Sondenrohrs (2);
- das Verbindungselement (18) erstreckt sich über mindestens ein Drittel der Länge des Sondenrohrs (2);
- der Filter (19,20) erstreckt sich über mindestens ein Viertel der Länge des Sondenrohrs (2);
- es sind mehrere Filter (19,20) hintereinander angeordnet;
- die Messkammer (33) grenzt in axialer Richtung an das von dem Verbindungselement (18) abgewandte Ende des Filters (19,20);
- die Messkammer (33) befindet sich an einem kalten Ende des Sondenrohrs (2), das dem freien Ende (12) gegenüberliegt;
- die Länge des Sondenrohrs (2) beträgt zwischen 2000 und 4000 mm;
- der Durchmesser des Sondenrohrs (2) beträgt zwischen 80 und 200 mm;
- die Breite des Ringspalts ist kleiner als 1 mm;
- die Breite des Ringraums (32) ist größer als 5 mm.

3. Gasentnahmesonde (1) nach einem der vorangehenden Ansprüche, **dadurch gekennzeichnet, dass** der mindestens eine rohrförmige Filter (19,20) von einem Druckrohr (30) durchragt wird, welches mindestens eine Durchlassöffnung (31) aufweist und dessen Innenraum mit der Messkammer (33) in Fluidverbindung steht.

4. Gasentnahmesonde (1) nach einem der vorangehenden Ansprüche, **dadurch gekennzeichnet, dass** die Ausstoßplatte (16) mit Zentrierkörpern (26) verbunden ist, die sich gegen die Wand des Innenmantels (14) abstützen.

5. Gasentnahmesonde (1) nach einem der vorangehenden Ansprüche, **dadurch gekennzeichnet, dass** die Ausstoßplatte (16) an einem Führungselement lösbar befestigt ist, in dem mindestens drei sich radial erstreckende Zentrierkörper (26) angeordnet sind.

6. Gasentnahmesonde (1) nach Anspruch 4 oder 5, **dadurch gekennzeichnet, dass** die Führungselemente austauschbar sind und aus einem weicheren Metall als der Innenmantel (14) bestehen.

7. Verfahren zur Messung der Zusammensetzung partikelbeladener Gase, bei dem:
- Messgas mittels eines von Kühlflüssigkeit durchströmten Sondenrohrs (2) mit einem Außenmantel (13) und einem Innenmantel (14) aus einer Gasströmung entnommen wird,
- das Messgas durch einen schmalen Ringspalt zwischen einer in dem Innenmantel (14) angeordneten Ausstoßplatte (16) und dem Innenmantel (14) im Bereich des freien Endes (12) des Sondenrohrs (2) in das Sondenrohr (2) einströmt,
- das Messgas durch einen rohrförmigen Filter (19,20) aus Sinterwerkstoff zu einer Messkammer (33) mit einem Messsensor geleitet wird, **dadurch gekennzeichnet, dass** das Messgas vor dem Einströmen in den Filter (19,20) durch einen zwischen dem Filter (19,20) und der Ausstoßplatte (16) angeordneten Ringraum (32) strömt, dessen radiale Erstreckung dem Mehrfachen der radialen Erstreckung des Ringspalts zwischen Ausstoßplatte (16) und Innenmantel (14) entspricht.

8. Verfahren nach Anspruch 7, **dadurch gekennzeichnet, dass** die Ausstoßplatte (16) in vorgegebenen Zeitinterwallen axial durch das freie Ende (12) aus dem Sondenrohr (2) herausbewegt wird, wobei ein Spülgas mit Überdruck in dem Innenmantel über die Oberfläche des Filters (19,20) und durch den Ringraum (32) geleitet wird und aus dem freien Ende (12) des Sondenrohrs (2) heraus strömt.

9. Verfahren nach Anspruch 7 oder 8, **dadurch gekennzeichnet, dass** eine Antriebsvorrichtung (10) das Sondenrohr (2) in vorgegebenen Zeitintervallen axial verschiebt.

10. Verfahren nach Anspruch 7, 8 oder 9, **dadurch gekennzeichnet, dass** eine Antriebsvorrichtung das Sondenrohr (2) in vorgegebenen Zeitintervallen dreht.

## Claims

1. A liquid-cooled gas sampling probe (1) comprising:
- a probe tube (2) through which cooling liquid flows and having an outer jacket (13) and an inner jacket (14) which are connected to one another in a sealed manner and form an enclosure of a flow space (15),
- at least one tubular filter (19, 20) made of sintered material, which is arranged inside the inner jacket (14) of the probe tube (2), wherein measuring gas entering the inner jacket (14) of the probe tube (2) through the free end (12) of the probe tube (2) flows through the tubular filter (19, 20) from the outside to the inside,
- at least one measuring chamber (33) with at least one measuring sensor, into which the measuring gas enters after flowing through the tubular filter (19, 20),
- an ejector plate (16) which is arranged in the region of the free end (12) of the probe tube (2) and is axially displaceable within the inner jacket (14), wherein during the measurement operation, a narrow annular gap between the exterior of the ejector plate (16) and the inner jacket (14) of the probe tube (2) allows measuring gas to flow into the probe tube (2),
**characterized in that** a connecting element (18) is arranged between the filter (19, 20) and the ejection plate (16), the diameter of the connecting element (18) being several millimeters smaller than the inner diameter of the inner jacket (14), so that an annular space (32) is formed between the connecting element (18) and the inner jacket (14), the radial extent of the annular space (32) corresponding to a multiple of the radial extent of the annular gap between the ejection plate (16) and the inner jacket (14).

2. The gas sampling probe (1) according to claim 1, **characterized in that** it has at least one of the following features:
- the length of the connecting element (18) is greater than the outer diameter of the probe tube (2);
- the connecting element (18) extends over at least one third of the length of the probe tube (2);
- the filter (19,20) extends over at least a quarter of the length of the probe tube (2);
- several filters (19,20) are arranged in series;
- the measuring chamber (33) is axially adjacent to the end of the filter (19, 20) remote from the connecting element (18);
- the measuring chamber (33) is located at a cold end of the probe tube (2) opposite the free end (12);
- the length of the probe tube (2) is between 2000 and 4000 mm;
- the diameter of the probe tube (2) is between 80 and 200 mm;
- the width of the annular gap is less than 1 mm;
- the width of the annular space (32) is more than 5 mm.

3. The gas sampling probe (1) according to one of the preceding claims, **characterized in that** the at least one tubular filter (19, 20) is traversed by a pressure tube (30) which has at least one passage opening (31) and whose interior is in fluid communication with the measuring chamber (33).

4. The gas sampling probe (1) according to one of the preceding claims, **characterized in that** the ejection plate (16) is connected to centering bodies (26) which bear against the wall of the inner jacket (14).

5. The gas sampling probe (1) according to one of the preceding claims, **characterized in that** the ejection plate (16) is detachably fixed to a guide element in which at least three radially extending centering bodies (26) are arranged.

6. The gas sampling probe (1) according to claim 4 or 5, **characterized in that** the guide elements are interchangeable and made of a softer metal than the inner jacket (14).

7. A method for measuring the composition of particle-laden gases, in which:
- measuring gas is extracted from a gas flow by means of a probe tube (2) through which cooling liquid flows and having an outer jacket (13) and an inner jacket (14),
- the measuring gas flows into the probe tube (2) through a narrow annular gap between an ejection plate (16) arranged in the inner jacket (14) and the inner jacket (14) in the region of the free end (12) of the probe tube (2),
- the measuring gas is conducted through a tubular filter (19, 20) of sintered material to a measuring chamber (33) having a measuring sensor,
**characterized in that**, before flowing into the filter (19, 20), the sample gas flows through an annular space (32) which is arranged between the filter (19, 20) and the ejection plate (16) and whose radial extent corresponds to the multiple of the radial extent of the annular gap between the ejection plate (16) and the inner jacket (14).

8. The method according to claim 7, **characterized in that** the ejection plate (16) is moved axially out of the probe tube (2) through the free end (12) at predetermined time intervals, wherein a purge gas with overpressure in the inner jacket is passed over the surface of the filter (19, 20) and through the annular space (32) and flows out of the free end (12) of the probe tube (2).

9. The method according to claim 7 or 8, **characterized in that** a drive device (10) axially displaces the probe tube (2) at predetermined time intervals.

10. The method according to claim 7, 8 or 9, **characterized in that** a drive device rotates the probe tube (2) at predetermined time intervals.

## Revendications

1. Sonde de prélèvement de gaz à refroidissement liquide (1), comprenant :
- un tube de sonde (2) parcouru par un liquide de refroidissement, comprenant une enveloppe extérieure (13) et une enveloppe intérieure (14) qui sont reliées l'une à l'autre de manière étanche et forment la limite d'un espace de circulation (15),
- au moins un filtre tubulaire (19, 20) en matériau fritté, qui est agencé dans l'enveloppe intérieure (14) du tube de sonde (2), du gaz de mesure entrant dans l'enveloppe intérieure (14) du tube de sonde (2) en passant par l'extrémité libre (12) du tube de sonde (2) parcourant le filtre tubulaire (19, 20) de l'extérieur vers l'intérieur,
- au moins une chambre de mesure (33) comprenant au moins un capteur de mesure, au sein de laquelle le gaz de mesure pénètre après avoir parcouru le filtre tubulaire (19, 20),
- une plaque de rejet (16), qui est agencée dans la région de l'extrémité libre (12) du tube de sonde (2) et peut être déplacée de manière axiale à l'intérieur de l'enveloppe intérieure (14), un interstice annulaire étroit entre le côté extérieur de la plaque de rejet (16) et l'enveloppe intérieure (14) du tube de sonde (2) permettant l'afflux de gaz de mesure dans le tube de sonde (2) lors de l'opération de mesure, **caractérisée en ce qu'**un élément de liaison (18), dont le diamètre est inférieur de plusieurs millimètres au diamètre intérieur de l'enveloppe intérieure (14), est agencé entre le filtre (19, 20) et la plaque de rejet (16), de sorte qu'un espace annulaire (32), dont l'étendue radiale correspond à un multiple de l'étendue radiale de l'interstice annulaire entre la plaque de rejet (16) et l'enveloppe intérieure (14), est créé entre l'élément de liaison (18) et l'enveloppe intérieure (14).

2. Sonde de prélèvement de gaz (1) selon la revendication 1, **caractérisée en ce qu'**elle présente au moins une des caractéristiques ci-dessous :
- la longueur de l'élément de liaison (18) est supérieure au diamètre extérieur du tube de sonde (2) ;
- l'élément de liaison (18) s'étend sur au moins un tiers de la longueur du tube de sonde (2) ;
- le filtre (19, 20) s'étend sur au moins un quart de la longueur du tube de sonde (2) ;
- plusieurs filtres (19, 20) sont agencés les uns derrière les autres ;
- la chambre de mesure (33) est contiguë dans la direction axiale à l'extrémité du filtre (19, 20) opposée à l'élément de liaison (18) ;
- la chambre de mesure (33) se trouve au niveau d'une extrémité froide, opposée à l'extrémité libre (12), du tube de sonde (2) ;
- la longueur du tube de sonde (2) est comprise entre 2 000 et 4 000 mm,
- le diamètre du tube de sonde (2) est compris entre 80 et 200 mm
- la largeur de l'interstice annulaire est inférieure à 1 mm,
- la largeur de l'espace annulaire (32) est supérieure à 5 mm.

3. Sonde de prélèvement de gaz (1) selon l'une quelconque des revendications précédentes, **caractérisée en ce que** le au moins un filtre tubulaire (19, 20) est traversé par un tube de pression (30) qui présente au moins un orifice de passage (31) et dont l'intérieur est en communication fluidique avec la chambre de mesure (33).

4. Sonde de prélèvement de gaz (1) selon l'une quelconque des revendications précédentes, **caractérisée en ce que** la plaque de rejet (16) est reliée à des corps de centrage (26) qui s'appuient contre la paroi de l'enveloppe intérieure (14).

5. Sonde de prélèvement de gaz (1) selon l'une quelconque des revendications précédentes, **caractérisée en ce que** la plaque de rejet (16) est fixée de manière amovible à un élément de guidage au sein duquel sont agencés au moins trois corps de centrage (26) s'étendant de manière radiale.

6. Sonde de prélèvement de gaz (1) selon la revendication 4 ou 5, **caractérisée en ce que** les éléments de guidage sont interchangeables et sont constitués d'un métal plus tendre que celui de l'enveloppe intérieure (14).

7. Procédé permettant de mesurer la composition de gaz chargés de particules, au cours duquel :
- du gaz de mesure est prélevé dans une circulation de gaz au moyen d'un tube de sonde (2) parcouru par un liquide de refroidissement et comprenant une enveloppe extérieure (13) et une enveloppe intérieure (14),
- le gaz de mesure afflue dans le tube de sonde (2) dans la région de l'extrémité libre (12) du tube de sonde (2) en passant par un interstice annulaire étroit situé entre une plaque de rejet (16), agencée dans l'enveloppe intérieure (14), et l'enveloppe intérieure (14),
- le gaz de mesure est dirigé à travers un filtre tubulaire (19, 20) en matériau fritté vers une chambre de mesure (33) comprenant un capteur de mesure,
**caractérisé en ce que** le gaz de mesure, avant d'affluer dans le filtre (19, 20), circule à travers un espace annulaire (32) qui est agencé entre le filtre (19, 20) et la plaque de rejet (16) et dont l'étendue radiale correspond à un multiple de l'étendue radiale de l'interstice annulaire entre la plaque de rejet (16) et l'enveloppe intérieure (14).

8. Procédé selon la revendication 7, **caractérisé en ce que** la plaque de rejet (16) est déplacée hors du tube de sonde (2) de manière axiale à travers l'extrémité libre (12) à des intervalles de temps prédéfinis, un gaz de rinçage en surpression étant dirigé dans l'enveloppe intérieure sur la surface du filtre (19, 20) et à travers l'espace annulaire (32) et s'écoulant par l'extrémité libre (12) du tube de sonde (2).

9. Procédé selon la revendication 7 ou 8, **caractérisé en ce qu'**un dispositif d'entraînement (10) déplace le tube de sonde (2) de manière axiale à des intervalles de temps prédéfinis.

10. Procédé selon la revendication 7, 8 ou 9, **caractérisé en ce qu'**un dispositif d'entrainement fait tourner le tube de sonde (2) à des intervalles de temps prédéfinis.
